# EUROPEAN PATENT SPECIFICATION

(11) **EP 1 045 692 B1**
(45) Date of publication and mention of the grant of the patent: **14.07.2004**
(21) Application number: 98960285.9
(22) Date of filing: 20.11.1998
(51) Int. Cl.: A61K 31/205, A61K 31/22, A61F 13/00, A61L 15/16

(54) **SKIN PROTECTION COMPOSITION**
ZUSAMMENSETZUNG ZUM SCHUTZ DER HAUT
COMPOSITION POUR LA PROTECTION DE LA PEAU

(30) Priority: 26.11.1997 EP 97120698
(43) Date of publication of application: 25.10.2000
(73) Proprietor: THE PROCTER & GAMBLE COMPANY, Cincinnati, Ohio 45202 (US)
(72) Inventor: PALUMBO, Gianfranco, D-61348 Bad Homburg (DE); GUARRACINO, Mario, I-64028 Silvi Marina (IT)
(74) Representative: McGregor, Judit Ester
(86) International application number: PCT/US1998/024692
(87) International publication number: WO 1999/026618

(56) References cited:
- WO-A-96/22347
- US-A- 4 780 249
- US-A- 5 561 106

## Description

The invention relates to cationic compounds and triester compounds to be used for preparation of compositions, which preferably can be in the form of lotions, creams, powders, oils, foams and the like, and be applied to the skin to prevent or reduce or treat skin rash or diaper rash resulting from lipolytic dermatitis. The compositions of the invention can also be applied to diapers, incontinent-pads, wipes and the like.

### Background

Skin rash caused by dermatitis, often referred to as diaper rash, has always been a problem encountered by the users of disposable absorbent articles, such as diapers, incontinence articles, sanitary towels, training pants etc Therefore. one of the biggest needs for these users is a solution to this type of skin rash problem.

The main factor which influences the development of skin rash is the contact of the skin with the wet body exudates. directly or for example contained in the absorbent article. Especially when the water content is high, skin rash can occur easily.

Manufacturers of diapers and skin care products have developed various products over the past decades which help reduce the occurrence of diaper rash (or skin rash).

The main focus thereby has been to reduce the exposure of the skin to the body exudates. This is for example done by introduction to the diaper of absorbing or better absorbing materials. The amount of water which is in contact with the skin is thus reduced.

Other products which are developed to address the skin-rash problem reduce the exposure of the skin to certain ingredients of the body exudates. An example of such ingredients of the exudate are bacteria which can infect the skin and thus start off or aggravate the skin rash.

For example, lotions have been developed which can form a barrier between the skin and the body exudates. Also, anti-inflammatory compositions can be applied to the skin or absorbent article.

EP 0191128 discloses a preparation comprising 8-hydroxy quinoline sulphate for treatment of skin irritation.

However, still one of the most heard complaints amongst users of absorbent articles such as diapers is the persistence of skin or diaper rash, despite the numerous products on the market which can be applied to prevent diaper or skin rash.

It has been discovered that yet another factor can set off or aggravate skin rash, namely the presence in the body exudate of various enzymes, especially lipase and protease enzymes.

EP 0117632B relates to disposable articles which comprise lipase inhibiting agents, preferably zinc containing components, and a vehicle material. Also mentioned is the use of glyceryl triacetate

US 3, 961,486 teaches the use of adipic acid to reduce the lipase enzyme activity and to reduce the skin rash.

When the skin is exposed to lipase enzymes, the lipids of the skin can be affected by these enzymes. Furthermore. the protease enzymes present in the body exudate on the skin can affect the proteins (or peptides) of the skin. The protection or barrier function of the top layer of the skin (the Strateum Corneum) will thus be diminished. This can effect the health of the skin and/or facilitate the infection of the skin. This can thus lead to skin or diaper rash.

It has been found that triester compounds similar to lipids or the lipids of the skin in particular can function as enzyme substrates, which, when acted upon by a hydrolysing esterase enzyme, such as lipase enzymes, will be hydrolysed resulting in the release of free acids. Firstly, the presence of these acids will lower the pH of the area where the esters where topical applied to. This will amount to inactivation of all or most enzymes present in this area, in the body exudates, such as the lipase enzymes, protease enzymes. Secondly, the esterase or lipase enzymes are 'de-activated', because rather than hydrolysing the esters, such as lipids, of the skin, they hydrolyse the alternative substrate. the triester compounds of the invention.

It is known that bile salts are present in the body exudates. These bile salts are known to emulsify the lipase enzymes in the body, which ensures that the lipase enzymes are capable of performing on the lipid-water interface. It has been found that these bile salts still have an emulsifying function once outside the body, in the body exudates. They aid the lipase enzyme which is present in the body exudate by attacking the lipids in the outer layer skin, exposed to the body exudate.

The inventors have found that these bile salts can be inactivated (and thereby the lipase can be deactivated) when the bile salts are reacted with specific cationic compounds. They have found that the compositions comprising the cationic compounds can thereto be applied directly to the skin. This can be done by introduction of the composition into a cosmetic cream, lotion, oil, powder. Alternatively, the composition can be applied to an absorbent article, such as a diaper, which then can be applied to the skin.

Surprisingly it has been found that the use of a combination of the cationic compounds and the triester compounds has an enhanced and elongated effect on the skin-rash or lipolytic dermatitis. Unexpectingly, it has been found that the inhibition or inactivation of the lipase enzymes by the inactivation of the bile salts by the cationic compounds does not make the triester compounds less useful as enzyme substrates. Namely, it has been found that the cationic compounds provide an immediate effect, i.e. inhibition or inactivation of the lipase enzymes, which may reduce over time, whilst the effect of the triester compounds is relatively delayed but long-lasting. Thus, the combination of the twocompounds provides an very effective, immediate and long-lasting reduction or prevention of the diaper/skin rash. resulting from dermatitis caused by the enzymes present on the body exudates on the skin.

### Summary of the Invention

The invention relates to the use of a compound (a) and a compound (b) for preparation of a composition for prevention, reduction or treatment of lipolytic dermatitis of the external skin, wherein (a) and (b) are:
(a) a triester compound of the formulation: 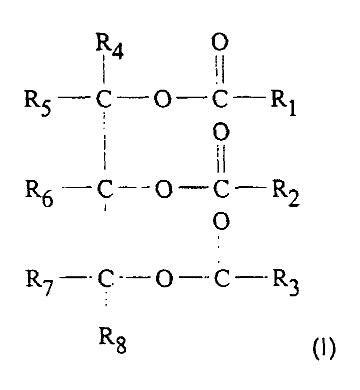 wherein R₁, R₂ and R₃ are independently an alkyl or alkenyl or hydroxyalkyl group with from 1 to 22 carbon atoms, and R₄, R₅, R₆, R₇ and R₈ are independently selected from the group consisting of C₁-C₁₀ linear or branched alkyl, alkenyl or hydroxyalkyl groups, hydroxy, chloride, bromide, amine or hydrogen ; and
(b) a cationic compound of the formulation: 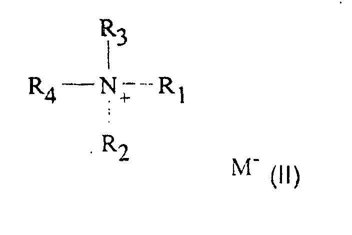 or 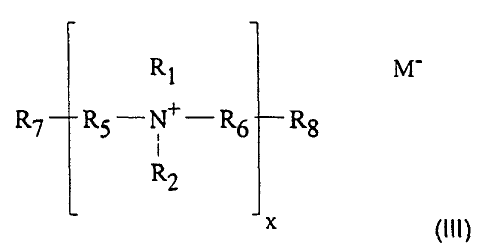 or an amphoteric compound and preferably an acidity source, the amphoteric compound having at its iso-electric point the formula: 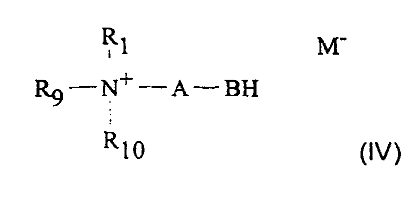
wherein R₁, R₂, R₃ and R₄ are independently selected from the group consisting of C₁-C₂₂ linear or branched alkyl, alkenyl, aryl, arylalkyl, amidoalkyl, (poly) alkoxy, hydroxyalkyl, or acyl groups, or two or more groups of R₁, R₂, R₃ and R₄ form together one or more ring structures; R₅, R₆ and A are independently selected from the group consisting of C₁-C₂₂ linear or branched alkylene, alkenylene, (poly) alkoxylene, hydroxyalkylene, arylalkylene or amido alkylene groups; R₇ and R₈ are independently an C₁-C₄ alkyl, alkenyl, alkoxy group or a hydroxy group or hydrogen; R₉ and R₁₀ are independently selected from the group consisting of C₁-C₂₂ linear or branched alkenyl, aryl, arylalkyl, amidoalkyl, (poly) alkoxy, hydroxyalkyl, or acyl groups, or two or more of the groups R₁, R₉ and R₁₀ form together one or more ring structures; BH is a proton donating group; x is from 2 to 4; and M- is a counter ion.

The invention also provides a disposable absorbent article, preferably a diaper or wipe, containing a composition which comprises the cationic compound and ester compound as described above, preferably at such a level that the compounds are each present at a level of from 0.01 % to 10% by weight of the article, preferably comprised in the topsheet of the diaper.

### Detailed Description of the Invention

The cationic compounds and triester compounds are used in for the preparation of compositions for prevention, reduction or treatment of lipolytic dermatitis of the (external) skin.

By treatment or reduction is meant herein the reduction of the lipolytic dermatitis or the rash of the skin, or diaper rash, which is caused by the lipase enzymes present on the skin, or at least stabilising the dermatitis or rash of the skin which is caused by these enzymes.

The composition of the invention can be applied to the skin which is in contact with at least the lipase enzymes. Such compositions can be comprised in a cream, lotion, oil, ointment, foam. powder or gel, which can be topical applied to the skin.

Alternatively, the compositions of the invention can be applied to an absorbent article, which can be brought in close contact with the skin which is in contact with the lipase enzymes Such articles are preferably disposable articles such as diapers, incontinent pads, training pants, sanitary towels, feminine hygiene garments, wet and dry wipes.

By the term "topical application" or "topical(ly) applied", as used herein, is meant directly laying on or spreading on epidermal tissue, especially outer skin.

The amount of the composition comprising the ester compounds of the invention will vary with the particular location of the condition being treated, the severity of the condition being treated, the expected duration of the treatment, any specific sensitivity to either the composition itself, or the concentration of the ester compounds specific to the user, the condition of the user, concurrent therapies being administered, other conditions present in the user.

For the present invention it is preferred that a minimum inhibitory concentration of the compositions containing the ester and cationic compounds of the invention is topically applied, to act as lipase de-activator or inhibitor to the area in need of treatment of the lypolytic dermatitis or the area where prevention of enzymatic dermatitis is desired in a form such that it is available to inhibit the activity of the lipase present.

This area (or "affected area", as used herein) is meant the area of the skin which is presently exhibiting any levels of skin rash or enzymatic dermatitis, or the area which will be in prolonged contact with body exudates containing the enzymes, in particular the lipase enzyme. This also includes the area immediately proximate to the described area. It is the area at which treatment, reduction of, and /or prevention is desired.

### Lipolytic dermatitis

This invention deals with compositions comprising the triester compounds and cationic compounds described above (or (disposable) absorbent articles incorporating the compositions) for the treatment of dermatitis, in particular diaper rash or diaper dermatitis caused by the lipase enzymes present in the body exudates (thus lipolytic dermatitis) and other conditions which are associated with prolonged contact of the skin with body exudates and/or the wearing of a absorbent article, or in particular a diaper.

Lipase, lipase enzyme or lipolytic enzyme is the trivial or common term employed to represent a group of enzymes belonging to the esterases. Their general activity is to hydrolyse fats present in the ester form (such as the glycerides found in human skin), and accordingly generate fatty acids and glycerol. Because this group of enzymes is so widely distributed in plants, moulds, bacteria, milk, and milk-products, as well as in almost all animal tissues, and because moreover human lipase enzymes are present in the pancreatic exudates, they are almost always present in body exudates.

The activity of lipase enzymes contributes to almost all skin rash, or in particular diaper rash, causing irritation by the digestive degenerative action of these enzymes on the skin per se and by breaking down the lipid- skin components, compromises the barrier property of the skin in the affected area. This breakdown of the integrity of the skin allows other components of the body exudates (urine and faeces in particular), which may not, by themselves, be irritating, to migrate through the compromised skin. At this point normally harmless components may then become irritating.

### Compositions

### Triester compounds

The present invention provides triester compounds, as defined above in formulation (I) and cationic compounds as defined above in formulations (II), (III), and (IV) for use in the preparation of compositions which can be used for treatment, prevention or reduction of the skin rash or particularly diaper rash, which is set off or aggravated by enzymes, in particular the esterase enzymes, lipase, and protease enzymes, thus being the result of enzymatic dermatitis.

The composition preferably comprise the triester compounds at a level of from 0.01% to 90%, more preferably from 0.5% to 60%, most preferably from 2% to 25% by weight of the composition.

It should be understood that for the purpose of this invention, the groups R₁-R₈ of formulations (I) and groups R₁-R₁₀ of formulations (II), (III) and (IV) above can be substituted by any appropriate substituent group.

Preferred triester compounds have one or more, preferably all of R₄, R₅, R₆, R₇ and
R₈ of said compound being hydrogen.

Preferably, the triester compounds has at least one, preferably all of R₁, R₂ and R₃
being C1-C4 alkyl groups.

It can be highly preferred that the triester compound is glycerol triacetate.

### Cationic compound

In the formulations (II), (III) and (IV) above, R₁, R₂, R₃ and R₉ are independently preferably C₁-C₈, more preferably C₁-C₄ alkenyl or alkoxy, more preferably alkyl groups, most preferably methyl or ethyl groups.

Preferably, R₄, R₅ and R₁₀ are independently C₈-C₁₈, more preferably C₁₂-C₁₆ alkenyl or alkoxy, more preferably alkyl or arylalkyl groups, whereby it can be preferred that one of the R₄, R₅ and R₉ substituents is benzyl group.

Alternatively, it can be preferred that the cationic compound comprises at least one R₁, R₂ or R₃ or R₉ being a poly alkoxy group. Thus, R₁, R₂, R₃ and R₉ preferably are independently polyalkoxy groups comprising C₂-C₆, preferably C₂-C₃ alkoxy units and having an alkoxylation number of from 2 to 50, preferably from 5 to 18. Then, R₃, R₄ and R₁₀ are independently preferably C₁-C₈, more preferably C₁-C₄ alkenyl or alkoxy , more preferably alkyl groups, most preferably methyl or ethyl groups.

A, R₅ and R₆ are, independently, preferably C₁-C₆ alkenylene or more preferably alkylene groups, most preferably methylene or ethylene.

Preferred compounds can be benzalkonium chloride or Merquat 2200 (Trade name, being a 2-Propeneamide polymer of N,N-dimethyl-N-2-Propenyl-1-amonium chloride).

Preferred cationic compounds of the formulas above comprise one or more substituted R₁, R₂, R₃,R₄, R₉ or R₁₀ groups and/ or a substituted R₅ and/or substituted R₆ group, whereby the substituent is selected from the group from the group consisting of derivatives of silicon, glucose. fructose and saccharose.
Preferred can be Glucquat 125 (trade name. being lauryl dimethyl glucet-10-hydroxydimonium chloride).

Preferred cationic compounds of the formula (III) above are betaine or sulpho betaine having preferably R₁ and R₉ being a methyl group.

The composition preferably comprise the cationic compounds at a level of from 0.01 % to 90%, more preferably from 0.5% to 60%, most preferably from 2% to 25% by weight of the composition.

### Lotions, creams, oils, ointments, foams, gels, powders and the like

The compositions in accordance with the invention can be cosmetic compositions, preferably in the form of lotions, creams, oils, foams, ointments, powders, gels and the like. They can comprise any of the ingredients commonly used in the art for such compositions.

It is to be understood that the ingredients of the compositions above will depend on the character of the composition, thus lotions will generally comprise different additional ingredients than powders.

In the cosmetic creams, lotions, gels. oils or powders comprising the composition of the invention preferably an acidity source is present, preferably such that is capable to reduce the pH of the skin to below a pH of 8, more preferably below a pH of 7, more preferably below a pH of 6, or even more preferred below a pH of 5.

A wide variety of optional ingredients such as non-occlusive moisturisers, humectants, gelling agents. neutralising agents, perfumes, colouring agents, can be added to the skin compositions herein.

It can be preferred that the cosmetic cream, lotion, gel, oil, ointment or powder are substantially free from nonionic and/ or anionic surfactants and/or phosphoric acid esters and/ or enzymes.

### Additional ingredients

The composition of the invention can comprise additional ingredients. Which ingredient are present and in which level depends on the character of the composition and the use thereof.

A highly preferred additional component to be used for the preparation of the compositions of the present invention or for use in the compositions of the present invention are certain additional ester compounds.

A highly preferred additional ester compound for use in, or for preparation of the compositions of the invention is of the formulation: 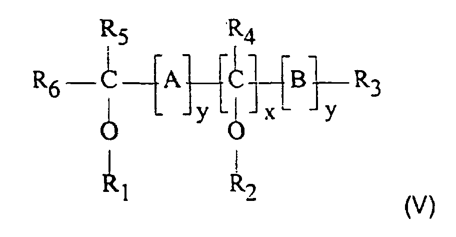 or 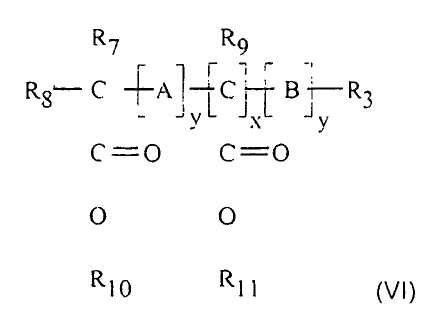 for preparation of a composition for prevention or treatment of enzymatic dermatitis wherein R₁ and each R₂ independently are an acyl group with from 2 to 22 carbon atoms, or an alkyl, alkenyl, arylalkyl, hydroxyalkyl group with from 1 to 24 carbon atoms or hydrogen, whereby at least one of R₁ and R₂ is such an acyl group, R₃ R₄, R₅, R₆, R₇, R₈, and R₉ are independently an alkyl, alkenyl, arylalkyl, hydroxyalkyl, alkoxy groups of from 1 to 24 carbon atoms, hydroxy group or hydrogen; R₁₀ and R₁₁ are independently an alkyl, alkenyl, arylalkyl, hydroxyalkyl, alkoxy groups of from 2 to 24 carbon atoms, hydroxy group or hydrogen; A and B are independently a C₁-C₆ linear or branched alkylene, alkenylene, alkoxylene, hydroxyalkylene groups; the values of x are independently from 0 to 15; the values of y are independently 0 or 1, with the proviso that when x =2 and y=0, at least one R₂ is an alkyl, alkenyl, arylalkyl, hydroxyalkyl group with from 1 to 24 carbon atoms or hydrogen

It should be understood that for the purpose of this invention, the groups R₁-R₁₁ of formulations (V) and (VI) above can be substituted by any appropriate substituent group.

Preferred are the ester compounds as defined above, wherein the compound is of formula (V) or (VI) wherein x is 1 or 2, y is 0; R₁ and one R₂ are a C₂-C₁₆ acyl group. R₁₀ and one or more R₁₁ are a C₂-C₁₆ alkyl group; R₃, R₄, R₅, R₆, R₇ and R₈ are hydrogen It is highly preferred that the additional ester compound is a mono or diester of formula (VI), most preferably a mono or diester of citric acid or tartaric acid (or salts thereof), or a triester of citric acid.

The additional ester compounds are preferably present in the compositions of the invention at a level of from 0.01% to 20%, more preferably from 0.05% to 10%, most preferably from 0.1% to 5% by weight of the composition.

### Process

Also encompassed in the invention is a process for reducing the enzyme activity of the enzymes present on the external skin, whereby the process comprises the steps of preferably topical applying a composition according to the invention to the external skin

Thereby, acids are formed (in situ), preferably capable of reducing the initial pH to below 7.9, more preferably below 7.5 or even 7.3.

It can be preferred that an additional acidity source is present, capable of reducing the pH to below 7.3, preferably below 6 or even 5.

The composition used in the process or the process is preferably such that within the first 15 minutes after application of the composition to the affected area, the lipase enzyme activity is reduced to 35%, preferably 25%. more preferably less than 20% of the initial lipase activity in this area.

Preferably the composition used in the process or the process is such that 60 minutes after application of the composition to the affected area, the lipase enzyme activity is still less than 45%, preferably 35%, more preferably less than 30% of the initial lipase activity in this area.

This process can be preferably done by applying an absorbent article which comprises the composition present in a suitable level, to the skin.

### Absorbent Articles

The compositions of the invention, comprising the triester compounds and the cationic compound, can be comprised in a absorbent article, preferably a disposable absorbent article. A particularly preferred absorbent articles therefor is a diaper, which preferably comprises the composition in the topsheet of the diaper.

As used herein, the term "absorbent articles" refers to devices which absorb and contain body exudates, and, more specifically, refers to devices which are placed against or in proximity to the body of the wearer to absorb and contain the various exudates discharged from the body The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner).

The structure of the disposable absorbent article is not critical to the practice of the present invention

Normally, the composition is incorporated into the absorbent article or diaper in particular in an amount which will deliver the required treatment or reduction or prevention of the lipolytic dermatitis preferably after frequent use.

The disposable absorbent article preferably contains the cationic compounds and the triester compounds in a compositions present at a level of from 0.01% to 30%, more preferably from 0.01% to 10%, most preferably from 0.05% to 5% by weight of the article.

An absorbent article generally comprises
- an absorbent core (which may consist of sub-structures);
- a fluid pervious topsheet;
- a fluid impervious backsheet;
- optionally further features like closure elements or elastification.

As used herein, the term "diaper" refers to an absorbent article generally worn by infants and incontinent persons that is worn about the lower torso of the wearer. It should be understood, however, that the present invention is also applicable to other absorbent articles such as incontinent briefs, incontinent undergarments, diaper holders and liners, feminine hygiene garments, and the like.

A preferred wipe for the purpose of this invention comprises an absorbent fibrous material or core into which the composition may be releasably incorporated. A highly preferred disposable wipe for the purposes of this invention compnses an absorbent fibrous material and a faeces-impermeable backing material; said backing being superposed or coextensive with one face of said absorbent fibrous material; said backing material most preferably being a web-backing material and most preferably having a width greater than said absorbent material providing side marginal portions which extend beyond said absorbent material, said margin portions being folded around and on top of the edges of said absorbent material. The compositions of the invention agent may be releasably incorporated into the wipe structure by diverse methods which will be readily apparent to those skilled in the art. For example, the compositions can be present in aqueous or volatile carrier such as water, ethanol, or the like, or creams. lotions, oils, ointments, gels or powders, and applied to the absorbent material by spraying, dipping, printing, soaking or otherwise contacting the absorbent material of the wipe with the lipase-inhibiting agent and its carrier. A skin cleansing agent, preferably an oleaginous cleansing agent, may optionally be releasably incorporated into the absorbent material as well.

The compositions of the present invention are preferably incorporated into a diaper, preferably into the absorbent core structure or most preferably into the topsheet structure. The composition may be incorporated into the diaper structure by diverse methods which will be readily apparent to those skilled in the art. For example, the composition can be, optionally after being dispersed aqueous or volatile carrier such as water, ethanol, or the like, applied to the diaper topsheet, to the absorbent core, or to the core side of the backsheet, by spraying, dipping, printing, soaking or otherwise contacting the selected structural element of the diaper with composition and optionally its carrier, which is called herein impregnation.

The diaper preferably comprises a liquid pervious topsheet, a liquid impervious backsheet joined with the topsheet, an absorbent core positioned between the topsheet and the backsheet. While the topsheet, the backsheet, and the absorbent core may be assembled in a variety of well known configurations, preferred diaper configurations are described generally in U.S Patent 3,860,003 entitled "Contractable Side Portions for Disposable Diaper" which issued to Kenneth B. Buell on January 14, 1975; and U S Patent Application Serial No. 07/715,152, allowed, "Absorbent Article With Dynamic Elastic Waist Feature Having A Predisposed Resilient Flexural Hinge", Kenneth B. Buell et al. filed June 13, 1991.

The backsheet is positioned adjacent the garment surface of the absorbent core and is preferably joined thereto by attachment means such as those well known in the art. For example, the backsheet may be secured to the absorbent core by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. The attachment means will preferably comprise an open pattern network of filaments of adhesive as is disclosed in U.S. Patent 4,573,986 entitled "Disposable Waste-Containment Garment", which issued to Minetola et al. on March 4, 1986, more preferably several lines of adhesive filaments swirled into a spiral pattern such as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Weremcz on June 27, 1989. Alternatively, the attachment means may comprise heat bonds, pressure bonds, ultrasonic bonds. dynamic mechanical bonds, or any other suitable attachment means or combinations of these attachment means as are known in the art.

The absorbent article may further comprise elastification or closure features well-known in the art and - for example - descnbed in E 0254476 (Alemany).

The topsheet is positioned adjacent the body surface of the absorbent core and is preferably joined thereto and to the backsheet by attachment means such as those well known in the art. As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element.

Generally, the topsheet is compliant, soft feeling, and non-irritating to the wearer's skin. Further, the topsheet is liquid pervious permitting liquids (e.g., urine) to readily penetrate through its thickness. A suitable topsheet may be manufactured from a wide range of materials, such as porous foams; reticulated foams; apertured plastic films; or woven or nonwoven webs of natural fibres (e.g., wood or cotton fibres), synthetic fibres (e.g., polyester or polypropylene fibres), or a combination of natural and synthetic fibres There are a number of manufacturing techniques which may be used to manufacture the topsheet. For example, the topsheet may be a nonwoven web of fibres spunbonded, carded, wet-laid, meltblown, hydroentangled, combinations of the above, or the like.

Preferably the topsheet comprises a means to adjust hydrophilicity of the material.

Absorbent cores comprise essentially all absorbent parts of the absorbent article other than the topsheet, which contribute to fluid absorbency or fluid handling.

The absorbent cores should be generally compressible, conformable, non-irritating to the wearer's skin, and capable of absorbing and retaining liquids such as urine and other certain body exudates.

The absorbent core can be made of a variety of materials Preferred materials are fibrous materials, which can form a fibrous web, natural occunng or synthetic fibres or optionally thermoplastic fibres. In addition thereto polymeric stiffening agents are preferably present. Also preferred can be hydrogel-forming polymers or absorbent polymers.

The various core, topsheet and backsheet materials can be arranged in any way known in the art, such as described in Weisman et al. (EP 0 202 125) or Alemany et al. (EP 0 254 476).

Also encompassed in the present invention is a process for making a diaper comprising the composition of the invention whereby the topsheet is impregnated with the composition before incorporation in the diaper..

## Claims

1. The use of a compound of (a) and a compound of (b) for preparation of a composition for prevention or treatment of lipolytic dermatitis of the external skin, wherein (a) and (b) are:
(a) a compound of the formulation: 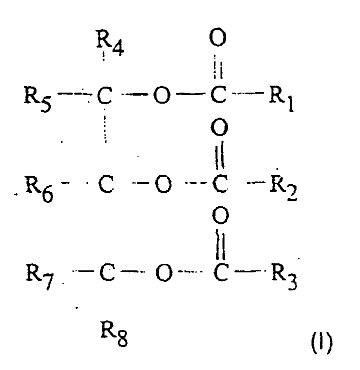 wherein R₁, R₂ and R₃ are independently an alkyl or alkenyl or hydroxyalkyl group with from 1 to 22 carbon atoms, and R₄, R₅, R₆, R₇ and R₈ are independently selected from the group consisting of C₁-C₁₀ linear or branched alkyl, alkenyl or hydroxyalkyl groups, hydroxy, chloride, bromide, amine or hydrogen ; and
(b) a cationic compound of the formulation: 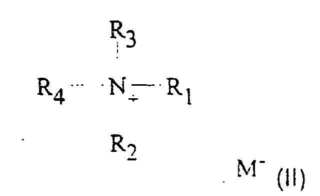 or 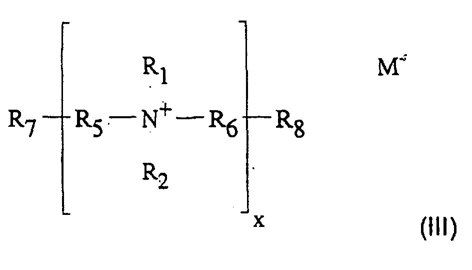 or an amphoteric compound and preferably an acidity source, the amphoteric compound having at its iso-electric point the formula: 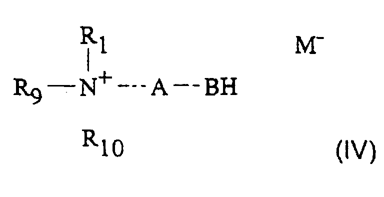
wherein R₁, R₂, R₃ and R₄ are independently selected from the group consisting of C₁-C₂₂ linear or branched alkyl, alkenyl, aryl, arylalkyl, amidoalkyl, (poly) alkoxy, hydroxyalkyl, or acyl groups, or two or more groups of R₁, R₂, R₃ and R₄ form together one or more ring structures; R₅, R₆ and A are independently selected from the group consisting of C₁-C₂₂ linear or branched alkylene, alkenylene, (poly) alkoxylene, hydroxyalkylene, arylalkylene or amido alkylene groups; R₇ and R₈ are independently an C₁-C₄ alkyl, alkenyl, alkoxy group or a hydroxy group or hydrogen; R₉ and R₁₀ are independently selected from the group consisting of C₁-C₂₂ linear or branched alkyl, alkenyl, aryl, arylalkyl, amidoalkyl, (poly) alkoxy, hydroxyalkyl, or acyl groups, or two or more of the groups R₁, R₉ and R₁₀ form together one or more ring structures; BH is a proton donating group; x is from 2 to 4; and M- is a counter ion.

2. The use of a compound (a) and (b) for preparation of a composition according to Claim 1, wherein the composition is for prevention or treatment of diaper rash.

3. The use of a compound (a) and (b) for preparation of a composition for prevention or treatment of lypolytic dermatitis of the external skin, according to Claim 1 or 2 wherein compound (a) has R₄, R₅, R₆, R₇ and R₈ being hydrogen.

4. The use of a compound (a) and (b) for preparation of a composition for prevention or treatment of lypolytic dermatitis of the external skin, according to any of Claim 1 to 3 wherein compound (a) has R₁, R₂ and R₃ being independently a C1-C4 alkyl group.

5. The use of a compound (a) and (b) for preparation of a composition for prevention or treatment of lypolytic dermatitis of the external skin, according to Claim 4 wherein the compound (a) is glycerol triacetate.

6. The use of a compound (a) and (b) for preparation of a composition for prevention or treatment of lypolytic dermatitis of the external skin, according to any preceding Claim wherein the cationic compound (b) comprises one or more substituted R₁, R₂, R₃,R₄, R₉ or R₁₀ groups and/ or a substituted R₅ and/or substituted R₆ group, whereby the substituent is selected from the group from the group consisting of derivatives of silicon, glucose, fructose and saccharose.

7. The use of a compound (a) and (b) for preparation of a composition for prevention or treatment of lypolytic dermatitis of the external skin, according to any preceding Claim wherein the cationic compound (b) comprises at least one R₁, R₂, R₃ and Rg being C₁-C₈, preferably C₁-C₄ alkyl, alkenyl or alkoxy groups, most preferably methyl or ethyl groups.

8. The use of a compound (a) and (b) for preparation of a composition for prevention or treatment of lypolytic dermatitis of the external skin, according to any of the preceding Claims, wherein the cationic compound (b) comprises at least one R₁, R₂ or R₃ or R₉ being a poly alkoxy group.

9. The use of a compound (a) and (b) for preparation of a composition for prevention or treatment of lypolytic dermatitis of the external skin, according to any of the preceding Claim, wherein the cationic compound (b) is of formula (IV), being a betaine compound or sulphobetaine compound.

10. A disposable absorbent article in the form of a wet-wipe, wipe, diaper or catamenial article for use on or application to the extemal skin containing the composition according to any preceding Claim.

11. A disposable absorbent article according to Claim 10 in the form of a diaper comprising the compositon, whereby the compound (a) and the compound (b) of the composition are present at a level of from 0.01% to 10% by weight of the diaper.

12. A disposable diaper according to Claim 11 whereby the diaper comprises a topsheet which contains the composition.

13. A process for making a diaper according to Claim 12 whereby the topsheet is impregnated with the composition before incorporation in the diaper.

## Revendications

1. Utilisation d'un composé (a) et d'un composé (b) pour la préparation d'une composition pour la prévention ou le traitement de la dermatite lipolytique de la peau externe, dans laquelle (a) et (b) sont :
(a) un composé de formule : 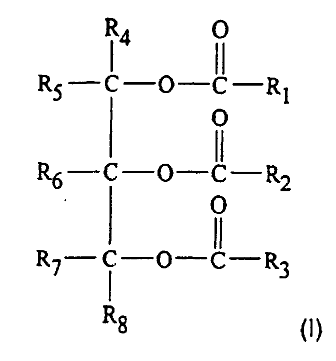 dans laquelle R₁, R₂ et R₃ sont indépendamment un radical alkyle ou alcényle ou hydroxyalkyle ayant de 1 à 22 atomes de carbone et R₄, R₅, R₆, R₇ et R₈ sont choisis indépendamment dans le groupe comprenant des radicaux hydroxyalkyle ou alcényle, alkyle, linéaires ou ramifiés en C₁ à C₁₀, un hydroxy, du chlore, du brome, une amine ou un hydrogène ; et
(b) un composé cationique de formule : 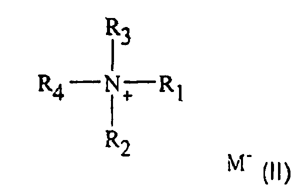
ou 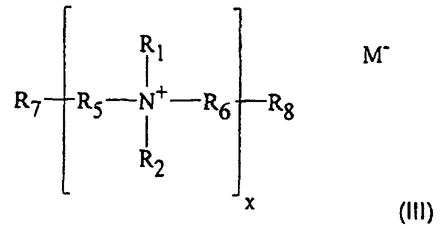 ou un composé amphotère et, de préférence, une source d'acidité, le composé amphotère ayant à son point isoélectrique la formule : 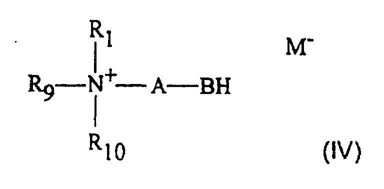 dans laquelle R₁, R₂, R₃ et R₄ sont choisis indépendamment parmi le groupe comprenant les radicaux acyle, hydroxyalkyle, (poly)alcoxy, amidoalkyle, arylalkyle, aryle, alcényle, alkyle linéaire ou ramifié en C₁ à C₂₂ ou deux ou plusieurs radicaux R₁, R₂, R₃ et R₄ forment conjointement une ou plusieurs structures cycliques, R₅, R₆ et A sont choisis indépendamment parmi le groupe comprenant les radicaux amindoalkylène, arylalkylène, hydroxyalkylène, (poly)alcoxylène, alcénylène, alkylène linéaires ou ramifiés en C₂₁ à C₂₂, R₇ et R₈ sont indépendamment un radical alcoxy, alcényle, alkyle en C₁-C₄ ou un groupe hydroxy ou de l'hydrogène, R₉ et R₁₀ sont choisis indépendamment parmi le groupe comprenant des radicaux acyle, hydroxyalkyle, (poly)alcoxy, amidoalkyle, arylalkyle, aryle, alcényle, alkyle linéaires ou ramifiés en C₁-C₂₂ ou deux ou plusieurs des radicaux R₁, R₉ et R₁₀ forment conjointement une ou plusieurs structures cycliques, BH est un groupe donneur de protons, x se situe de 2 à 4 et M- est un contre-ion.

2. Utilisation d'un composé (a) et (b) pour la préparation d'une composition selon la revendication 1, dans laquelle la composition est destinée à empêcher ou à traiter l'érythème fessier.

3. Utilisation d'un composé (a) et (b) pour la préparation d'une composition destinée à empêcher ou à traiter la dermatite lipolytique de la peau externe selon la revendication 1 ou 2, dans laquelle dans le composé (a) R₄, R₅, R₆, R₇ et R₈ sont de l'hydrogène.

4. Utilisation d'un composé (a) et (b) pour la préparation d'une composition destinée à empêcher ou à traiter la dermatite lipolytique de la peau externe selon l'une quelconque des revendications 1 à 3, dans laquelle dans le composé (a) R₁, R₂ et R₃ sont indépendamment un radical alkyle en C₁-C₄.

5. Utilisation d'un composé (a) et (b) pour la préparation d'une composition destinée à empêcher ou à traiter la dermatite lipolytique de la peau externe selon la revendication 4, dans laquelle le composé (a) est du triacétate de glycérol.

6. Utilisation d'un composé (a) et (b) pour la préparation d'une composition destinée à empêcher ou à traiter la dermatite lipolytique de la peau externe selon l'une quelconque des revendications précédentes, dans laquelle le composé cationique (b) comprend un ou plusieurs radicaux substitués R₁, R₂, R₃, R₄, R₉ ou R₁₀ et/ou un radical R₅ substitué et/ou un radical R₆ substitué, dans laquelle le substituant est choisi à partir du groupe comprenant des dérivés de saccharose, de fructose, de glucose et de silicone.

7. Utilisation d'un composé (a) et (b) pour la préparation d'une composition destinée à empêcher ou à traiter la dermatite lipolytique de la peau externe selon l'une quelconque des revendications précédentes, dans laquelle le composé cationique (b) comprend au moins un radical R₁, R₂, R₃ et R₉ qui est un radical alcoxy, alcényle, alkyle en C₁-C₈, de préférence en C₁-C₄, de préférence des radicaux méthyle ou éthyle.

8. Utilisation d'un composé (a) et (b) pour la préparation d'une composition destinée à empêcher ou à traiter la dermatite lipolytique de la peau externe selon l'une quelconque des revendications précédentes, dans laquelle le composé cationique (b) comprend au moins un radical R₁, R₂ ou R₃ ou R₉ qui est un radical polyalcoxy.

9. Utilisation d'un composé (a) et (b) pour la préparation d'une composition destinée à empêcher ou à traiter la dermatite lipolytique de la peau externe selon l'une quelconque des revendications précédentes, dans laquelle le composé cationique (b) est de formule (IV) qui est un composé de bétaïne ou un composé de sulfobétaïne.

10. Article absorbant jetable sous la forme d'un article cataménial, d'une couche, d'une lingette, d'une lingette humide destinée à être utilisée ou à être appliquée sur la peau externe, renfermant la composition selon l'une quelconque des revendications précédentes.

11. Article absorbant jetable selon la revendication 10, sous la forme d'une couche, renfermant la composition, dans lequel le composé (a) et le composé (b) de la composition sont présents à raison de 0,01 % à 10 % en poids de la couche.

12. Couche jetable selon la revendication 11, dans laquelle la couche comprend une feuille de dessus qui renferme la composition.

13. Procédé pour fabriquer une couche selon la revendication 12, dans lequel la feuille de dessus est imprégnée avec la composition avant l'incorporation dans la couche.

## Patentansprüche

1. Verwendung einer Verbindung aus (a) und einer Verbindung aus (b) zur Herstellung einer Zusammensetzung zur Verhinderung oder Behandlung lipolytischer Dermatitis der äußeren Haut, worin (a) und (b) wie folgt sind:
(a) eine Verbindung der Formel 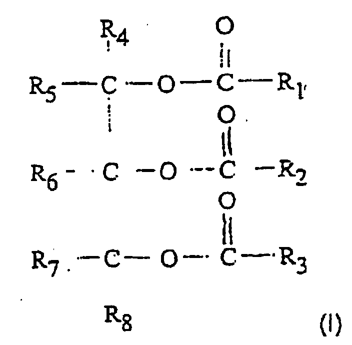 worin R₁, R₂ und R₃ unabhängig eine Alkyl- oder Alkenyl- oder Hydroxyalkylgruppe mit 1 bis 22 Kohlenstoffatomen sind, und R₄, R₅, R₆, R₇ und R₈ unabhängig voneinander gewählt sind aus der Gruppe, bestehend aus linearen oder verzweigten C₁-C₁₀-Alkyl-, -Alkenyl- oder -Hydroxyalkylgruppen, Hydroxy, Chlorid, Bromid, Amin oder Wasserstoff; und
(b) eine kationische Verbindung der Formel: 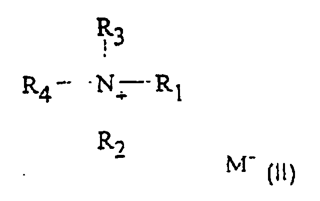 oder 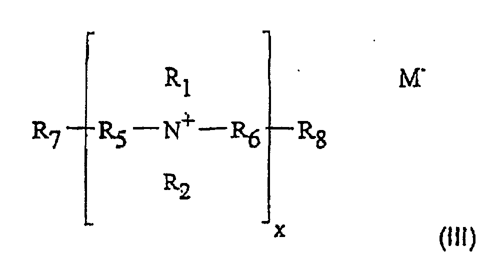 oder eine amphotere Verbindung und vorzugsweise eine Aciditätsquelle, wobei die amphotere Verbindung an ihrem isoelektrischen Punkt die Formel besitzt: 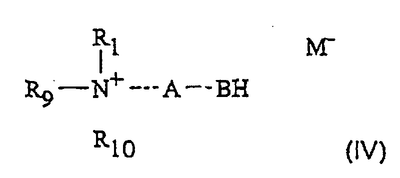
worin R₁, R₂, R₃ und R₄ unabhägig voneinander gewählt sind aus der Gruppe, bestehend aus linearen oder verzweigten C₁-C₂₂-Alkyl-, -Alkenyl-, Aryl-, Arylalkyl-, Amidoalkyl-, (Poly)alkoxy-, Hydroxyalkyl- oder Acylgruppen, oder zwei oder mehrere Gruppen aus R₁, R₂, R₃ und R₄ zusammen eine oder mehrere Ringstrukturen bilden; R₅, R₆ und A unabhängig voneinander gewählt sind aus der Gruppe, bestehend aus linearen oder verzweigten C₁-C₂₂-Alkylen-, Alkenylen-, Polyalkoxylen-, Hydroxyalkylen-, Arylalkylen- oder Amidoalkylengruppen; R₇ und R₈ unabhängig voneinander eine C₁-C₄-Alkyl-, Alkenyl-, Alkoxygruppe oder eine Hydroxygruppe oder Wasserstoff sind; R₉ und R₁₀ unabhängig voneinander gewählt sind aus der Gruppe, bestehend aus linearen oder verzweigten C₁-C₂₂-Alkyl-, Alkenyl-, Aryl-, Arylalkyl-, Amidoalkyl-, Polyalkoxy-, Hydroxyalkyl- oder Acylgruppen, oder zwei oder mehrere der Gruppen R₁, R₉ und R₁₀ zusammen eine oder mehrere Ringstrukturen bilden; BH eine Protonendonatorgruppe ist; x 2 bis 4 ist; und M- ein Gegenion ist.

2. Verwendung einer Verbindung (a) und (b) zur Herstellung einer Zusammensetzung nach Anspruch 1, wobei die Zusammensetzung zur Verhinderung oder Behandlung von Windelekzem vorgesehen ist.

3. Verwendung einer Verbindung (a) und (b) zur Herstellung einer Zusammensetzung zur Verhinderung oder Behandlung lipolytischer Dermatitis der äußeren Haut nach Anspruch 1 oder 2, wobei bei der Verbindung (a) R₄, R₅, R₆, R₇ und R₈ Wasserstoff sind.

4. Verwendung einer Verbindung (a) und (b) zur Herstellung einer Zusammensetzung zur Verhinderung oder Behandlung lipolytischer Dermatitis der äußeren Haut nach mindestens einem der Ansprüche 1 bis 3, wobei bei der Verbindung (a) R₁, R₂ und R₃ unabhängig voneinander eine C₁-C₄-Alkylgruppe sind.

5. Verwendung einer Verbindung (a) und (b) zur Herstellung einer Zusammensetzung zur Verhinderung oder Behandlung lipolytischer Dermatitis der äußeren Haut nach Anspruch 4, wobei die Verbindung (a) Glycerintriacetat ist.

6. Verwendung einer Verbindung (a) und (b) zur Herstellung einer Zusammensetzung zur Verhinderung oder Behandlung lipolytischer Dermatitis der äußeren Haut nach mindestens einem vorangehenden Anspruch, wobei die kationische Verbindung (b) eine oder mehrere substituierten R₁-, R₂-, R₃-, R₄-, R₉- oder R₁₀-Gruppen und/oder eine substituierte R₅- und/oder substituierte R₆-Gruppe umfasst, wobei der Substituent aus der Gruppe gewählt ist, bestehend aus Derivaten von Silicium, Glucose, Fructose und Saccharose.

7. Verwendung einer Verbindung (a) und (b) zur Herstellung einer Zusammensetzung zur Verhinderung oder Behandlung lipolytischer Dermatitis der äußeren Haut nach mindestens einem vorangehenden Anspruch, wobei bei der kationischen Verbindung (b) mindestens eines von R₁, R₂, R₃ und R₉ C₁-C₈-, vorzugsweise C₁-C₄-Alkyl, -Alkenyl- oder Alkoxygruppen, am meisten bevorzugt Methyl- oder Ethylgruppen sind.

8. Verwendung einer Verbindung (a) und (b) zur Herstellung einer Zusammensetzung zur Verhinderung oder Behandlung lipolytischer Dermatitis der äußeren Haut nach mindestens einem der vorangehenden Ansprüche, wobei bei der Verbindung (b) mindestens eines von R₁, R₂ oder R₃ oder R₉ eine Polyalkoxygruppe ist.

9. Verwendung einer Verbindung (a) und (b) zur Herstellung einer Zusammensetzung zur Verhinderung oder Behandlung lipolytischer Dermatitis der äußeren Haut nach mindestens einem vorangehenden Anspruch, wobei die kationische Verbindung (b) der Formel (IV) entspricht, welche eine Beatinverbindung oder Sulfobetainverbindung ist.

10. Wegwerfbarer absorbierender Gegenstand in Form eines Feuchtwischtuchs, Wischtuchs, Windel oder Menstruationsartikels zur Verwendung auf oder Aufbringung auf die äußere Haut, enthaltend die Zusammensetzung nach mindestens einem vorangehenden Anspruch.

11. Wegwerfbarer absorbierender Gegenstand nach Anspruch 10 in Form einer Windel, umfassend die Zusammensetzung, wobei die Verbindung (a) und die Verbindung (b) der Zusammensetzung in einem Anteil von 0,01 bis 10 Gew.-% der Windel vorliegen.

12. Wegwerfwindel nach Anspruch 11, wobei die Windel eine Oberlage umfasst, welche die Zusammensetzung enthält.

13. Verfahren zur Herstellung einer Windel nach Anspruch 12, wobei die Oberlage mit der Zusammensetzung imprägniert wird, bevor sie in die Windel eingearbeitet wird.
